(19) Europäisches Patentamt
European Patent Office
Office européen des brevets

(11) **EP 1 921 060 B1**

(12) **EUROPÄISCHE PATENTSCHRIFT**

(45) Veröffentlichungstag und Bekanntmachung des Hinweises auf die Patenterteilung:
**03.03.2010 Patentblatt 2010/09**

(51) Int Cl.:
*C07C 209/84* (2006.01)     *C07C 209/36* (2006.01)

(21) Anmeldenummer: **07021397.0**

(22) Anmeldetag: **02.11.2007**

(54) **Verfahren zur Entfernung von Verbindungen mit phenolischen Hydroxygruppen aus aromatischen Aminen**

Procedure for the removal of compounds with phenolic hydroxy groups from aromatic amines

Procédure d'enlèvement des composés avec les groupes hydroxy phénoliques des amines aromatiques

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **10.11.2006 DE 102006052989**

(43) Veröffentlichungstag der Anmeldung:
**14.05.2008 Patentblatt 2008/20**

(73) Patentinhaber: **Bayer MaterialScience AG**
**51368 Leverkusen (DE)**

(72) Erfinder:
• **Sommer, Knut, Dr.**
**47798 Krefeld (DE)**

• **Dugal, Markus, Dr.**
**47906 Kempen (DE)**
• **Schwarz, Ido, Dr.**
**40474 Düsseldorf (DE)**
• **Wershofen, Stefan, Dr.**
**41065 Mönchengladbach (DE)**
• **Lehner, Peter, Dr.**
**40878 Ratingen (DE)**
• **Schubert, Stephan, Dr.**
**51375 Leverkusen (DE)**
• **Grosse Böwing, Alexandra, Dr.**
**50935 Köln (DE)**

(56) Entgegenhaltungen:
**JP-A- 49 035 341    SU-A1- 592 436**

EP 1 921 060 B1

**Beschreibung**

Die Erfindung betrifft ein Verfahren zur Entfernung von Verbindungen mit phenolischen Hydroxygruppen aus einem mindestens ein aromatisches Amin enthaltenden Gasstrom, welcher aus der Gasphasen-Hydrierung der entsprechenden aromatischen Nitroverbindungen mit Wasserstoff hervorgeht, durch Adsorption an einem basischen Feststoff. Verbindungen mit phenolischen Hydroxygruppen sind solche, die mindestens eine Hydroxygruppe an einem aromatischen Ring tragen. Im Fall der Herstellung von Anilin und der Reinigung des Anilins ist die phenolische Hydroxygruppen tragende Verunreinigung im Wesentlichen das Phenol selbst, aber auch solche Derivate, die zusätzlich zur OH-Funktion noch weitere funktionelle Gruppen tragen, wie etwa die verschiedenen Aminophenole.

Der basische Feststoff kann aufgrund seiner basischen Eigenschaften Verbindungen mit phenolischen Hydroxygruppen aufgrund deren saurer Eigenschaften bevorzugt adsorbieren und nach bevorzugt vollständiger Beladung anschließend regeneriert werden. Die Regeneration kann z. B. durch Behandlung mit Sauerstoff oder sauerstoffhaltigen Gasgemischen bei erhöhter Temperatur erfolgen. Die Adsorption kann in einem Festbett mit ausreichend großer Kapazität oder unter Betrieb mehrerer Festbetten im Wechsel erfolgen, wobei die jeweils nicht genutzten Festbetten regeneriert werden können.

Aromatische Amine sind wichtige Zwischenprodukte, welche preiswert und in großen Mengen zur Verfügung stehen müssen. Daher müssen z. B. für die Hydrierung von Nitrobenzol zu Anilin Anlagen mit sehr großen Kapazitäten gebaut werden. Anilin ist wichtiges Zwischenprodukt z. B. zur Herstellung von Methylendiphenyldiisocyanat (MDI) und wird im großtechnischen Maßstab in der Regel durch katalytische Hydrierung von Nitrobenzol mit Wasserstoff hergestellt (siehe z. B. DE-OS 2201528, DE-OS 3414714, US 3136818, EP 0 696 573 B1, EP 0 696 574 B1). Bei dieser Reaktion werden neben dem Zielprodukt Anilin auch Nebenkomponenten mit phenolischen Hydroxygruppen, wie z. B. Phenol selbst oder Aminophenole gebildet, die vor einer weiteren Anwendung des. Anilins in Folgeprozessen durch Destillation entfernt werden müssen. Insbesondere die Trennung von Phenol und Anilin stellt aufgrund der eng zusammen liegenden Siedepunkte für die Destillationstechnik eine große Herausforderung dar, was sich in der Verwendung langer Destillationskolonnen mit großer Trennstufenzahl und hoher Rücklaufverhältnisse mit entsprechend hohem Investitions- und Energieaufwand widerspiegelt.

Neuere Ansätze beschreiben den Einsatz von löslichen Alkalien zur Extraktion oder einen Alkalizusatz bei der Destillation. So wird in JP-A-49-035341 ein alternatives Verfahren beschrieben, bei dem das Rohanilin mit festen Alkalien (z. B. festes Natriumhydroxid) in einem Festbett in Kontakt gebracht und erst anschließend in die Destillation geleitet wird, bzw. bei dem die Destillation in Gegenwart der festen Alkalie in Konzentrationen von 0,1 - 3 Massen-% bezogen auf die zu destillierende Anilin-Menge durchgeführt wird. Hierdurch wird die Abtrennung farbkritischer Komponenten wie Aminophenole vereinfacht. Nachteilig bei diesem Verfahren ist jedoch der Einsatz von hohen molaren Überschüssen der festen Alkalien in Bezug auf die zu entfernenden sauren Nebenkomponenten bzw. die Unmöglichkeit der genauen Dosierung der alkalischen Verbindungen. Dies kann einerseits bei Überdosierung zu Korrosionsproblemen, Ausfällungen und hochviskosen Sumpfphasen in der Destillationskolonne und andererseits bei Unterdosierung zu einer unvollständigen Entfernung der kritischen Komponenten führen.

JP-A-08-295654 beschreibt als Alternative zur destillativen Entfernung von Verbindungen mit phenolischen Hydroxygruppen aus Anilin eine Extraktion mit verdünnter wässriger Natronlauge (oder Kalilauge), wodurch das Phenol als Natriumphenolat größtenteils in die wässrige Phase transferiert wird, die durch die anschließende Phasentrennung als obere Phase abgetrennt wird. Für eine effektive Reduzierung des Phenolgehaltes ist ein molares Verhältnis NaOH : Phenol im Bereich von 3 : 1 - 100 : 1 erforderlich. Nachteilig bei diesem Verfahren ist ein hoher NaOH-Verbrauch (molare Überschüsse), das Anfallen eines alkaliphenolathaltigen Abwassers, was zu zusätzlichen Entsorgungskosten führt, sowie ein zusätzlicher Investitionsaufwand für die Extraktion.

Die Aufgabe der vorliegenden Erfindung war daher, ein einfaches und wirtschaftliches Verfahren zur Aufreinigung von aromatischen Aminen, die durch katalytische Hydrierung der entsprechenden Nitroverbindungen hergestellt werden, zur Verfügung zu stellen, bei dem auf eine aufwändige Destillation verzichtet und gleichzeitig die Größe der Abwasserströme deutlich reduziert werden kann.

Die Erfindung betrifft ein Verfahren zur Entfernung von Verbindungen mit phenolischen Hydroxygruppen aus einem mindestens ein aromatisches Amin enthaltenden Gasstrom, welcher aus der Gasphasen-Hydrierung der entsprechenden aromatischen Nitroverbindungen mit Wasserstoff hervorgeht, bei dem die Verbindungen mit phenolischen Hydroxygruppen an einem basischen Feststoff adsorbiert werden.

In dem erfindungsgemäßen Verfahren werden Verbindungen mit phenolischen Hydroxygruppen aufgrund ihrer sauren Eigenschaften an dem basischen Feststoff adsorbiert. Bevorzugt weist der über den basischen Feststoff geleitete Gasstrom eine Eingangstemperatur zwischen 150 und 500 °C und einen absoluten Druck von 1 bis 50 bar auf. Der basische Feststoff kann nach vollständiger Beladung durch Behandlung mit Sauerstoff oder sauerstoffhaltigen Gasgemischen bei erhöhter Temperatur regeneriert werden. Die Adsorption erfolgt bevorzugt entweder in *einem* Festbett mit ausreichend großer Kapazität oder unter Betrieb *mehrerer* Festbetten *im Wechsel,* wobei die jeweils nicht genutzten Festbetten dann bevorzugt regeneriert werden.

Der eingesetzte mindestens ein aromatisches Amin enthaltende Gasstrom kann aus allen technisch üblichen Verfahren zur Gasphasen-Hydrierung von aromatischen Nitroverbindungen stammen. Bevorzugt wird die Hydrierung an ortsfesten, heterogenen Trägerkatalysatoren, wie z. B. Pd auf Aluminiumoxid oder Kohleträgern, in Festbettreaktoren bei einem absoluten Druck von 1 - 50 bar und einer Temperatur im Bereich von 150-600 °C unter adiabaten Bedingungen in Kreisgasfahrweise, d. h. unter Rückführung von während der Hydrierung nicht umgesetzten Wasserstoffs, durchgeführt. Das Verfahren ist beispielsweise in EP 0 696 573 B1 und EP 0 696 574 B1 beschrieben.

Bevorzugtes aromatisches Amin ist Anilin.

Als basische Feststoffe können alle Brønstedt-basischen anorganischen Verbindungen von Elementen der Gruppen 1 bis 2 und 12 bis 14 des Periodensystems der Elemente (Nomenklatur nach IUPAC-Empfehlung von 1986) eingesetzt werden, insbesondere die Oxide und Hydroxide genannter Elemente, wobei auch beliebige Kombinationen möglich sind. Bevorzugt sind MgO, Hydrotalcite und basisches $Al_2O_3$, besonders bevorzugt ist basisches $Al_2O_3$. Die basischen anorganischen Verbindungen können entweder als solche oder auf einem geeigneten Träger vorliegen. Als Träger kommen alle in der heterogenen Hydrierkatalyse gängigen Materialien in Frage, z. B. die in EP 0 696 573 B1 und EP 0 696 574 B1 genannten, besonders geeignet sind α-Aluminiumoxide und Graphite bzw. graphithaltige Kohleträger.

Basische Zeolithe können ebenfalls als basische Feststoffe eingesetzt werden. Solche Zeolithe werden beispielsweise in den folgenden Publikationen beschrieben:

Rep; M.; Palomares, A. E.; Eder-Mirth, G.; van Ommen, J. G.; Rösch, N.; Lercher, J. A., "Interaction of Methanol with Alkali Metal Exchanged Molecular Sieves. 1. IR Spectroscopic Study", Journal of Physical Chemistry B (2000), 104 (35), 8624 - 8630
oder

Tsyganenko, A. A.; Kondratieva, E. V.; Yanko, V. S.; Storozhev, P. Yu., "FTIR study of CO adsorption on basic zeolites", Journal of Materials Chemistry (2006), 16 (24), 2358 - 2363;
oder

Plant, David F.; Simperler, Alexandra; Bell, Robert G., "Adsorption of methanol on zeolites X and Y. An atomistic and quantum chemical study", Journal of Physical Chemistry B (2006), 110 (12), 6170 - 6178.

Bevorzugt sind $Cs_mX$, $Cs_mY$, $Cs_mNa_nX$, $Cs_mNa_nY$, $Rb_mX$ und $Rb_mY$, worin die Stöchiometriekoeffizienten "m" und "n" unter der Randbedingung, dass die Summe aus beiden die negative Ladung von X bzw. Y ausgleichen muss (Elektroneutralitätsbedingung), jeden beliebigen Wert (auch Null) annehmen können. Solche Zeolithe sind im Allgemeinen wasserhaltig; der Einfachheit halber wurde das Wasser in obigen Formeln nicht aufgeführt. Die Definitionen der Bezeichnungen "X" und "Y" sind in Wiberg, N., "Hollemann-Wiberg - Lehrbuch der Anorganischen Chemie", 101. Auflage, Walter de Gruyter, Berlin, New York 1995, S. 939, Anmerkung 166 enthalten, worauf hiermit Bezug genommen wird.

Die eingesetzten basischen Feststoffe zeigen bevorzugt eine sehr hohe Adsorptionsselektivität bzgl. Verbindungen mit phenolischen Hydroxygruppen, wobei unter der *Adsorptionsselektivität A* der Ausdruck

$$A = \frac{B}{B + {x_B}/{x_C} \cdot C} \cdot 100\,\%$$

verstanden wird, und worin die verwendeten Symbole folgende Bedeutungen haben:

$B$ = Mole adsorbierter Verbindungen mit phenolischen Hydroxygruppen pro kg basischer Feststoff,

$C$ = Mole adsorbierten aromatischen Amins pro kg basischer Feststoff,

$x_B/x_C$ = Gewichtungsfaktor, der den hohen Amin-Überschuss berücksichtigt,

$x_B$ = Molares Verhältnis (Molenbruch) der Gehalte der Verbindungen mit phenolischen Hydroxygruppen zur Summe der Gehalte der Verbindungen mit phenolischen Hydroxygruppen und Amin im Einsatzgemisch,

$x_C$ = Molares Verhältnis (Molenbruch) der Gehalte des Amins zur Summe der Gehalte der Verbindungen mit phenolischen Hydroxygruppen und Amin im Einsatzgemisch.

Dabei wird die Adsorptionsselektivität experimentell bestimmt, indem durch ein von außen auf 240 °C beheiztes Adsorberrohr von 60 cm Länge und 2,5 cm Durchmesser, gefüllt mit Glaskugeln und einer Schüttung aus 14 g des Adsorbens, bei Umgebungsdruck ein gasförmiger Strom aus 49,12 g/h Anilin (dotiert mit 500 ppm Phenol), 20,1 g/h Wasser und 24,5 l/h Wasserstoff geleitet wird. Der aus dem Adsorber austretende Gasstrom wird bei 3 °C in einem Auffangbehälter kondensiert und der Phenolgehalt gaschromatographisch bestimmt. Das Adsorbens wird separat in einem Soxhlet-Extraktor für 4 h mit Isopropanol extrahiert, und der Anilin- sowie Phenol-Gehalt des Destillats werden gaschromatographisch bestimmt.

Bevorzugt eingesetzte basische Feststoffe zeigen eine Adsorptionsselektivität A von > 90%, besonders bevorzugt > 98%, ganz besonders bevorzugt > 99,5 %. Es werden bevorzugt MgO und besonders bevorzugt basische Aluminiumoxide als Adsorptionsmittel eingesetzt.

Der über den basischen Feststoff geleitete Gasstrom, der das zu reinigende Amin enthält, hat bevorzugt eine Eingangstemperatur zwischen 150 und 500 °C, besonders bevorzugt zwischen 200 und 300°C, ganz besonders bevorzugt zwischen 220 und 280°C und einen absoluten Druck von bevorzugt 1 bis 50 bar, besonders bevorzugt 2 bis 20 bar, ganz besonders bevorzugt 2 bis 10 bar. In einer besonders günstigen Ausführungsform des Verfahrens wird unter Bedingungen gearbeitet, die von den Erfordernissen der Hydrierreaktion vorgegebenen sind, beispielsweise bei der Temperatur und dem Druck des Produktgasgemisches nach Verlassen des letzten Wärmetauschers in einer Produktionsstraße.

In einer besonders wirtschaftlichen Ausführungsform des erfindungsgemäßen Verfahrens ist der basische Feststoff identisch mit dem Träger oder auf dem Träger aufgebracht, der auch als Träger für die hydrieraktiven Metalle in der Hydrierung der Nitroverbindung eingesetzt wird. In einem solchen Fall ist ein nachgeschaltetes separates Festbett mit einem zusätzlichen basischen Feststoff überflüssig.

Dann kann entweder ein Träger eingesetzt werden, der von vorneherein die gewünschten basischen Eigenschaften besitzt. Oder es wird ein Träger eingesetzt, der vor oder bevorzugt nach der Belegung mit den hydrieraktiven Metallen durch Aufbringen basischer anorganischer Verbindungen modifiziert wurde.

Wenn die Adsorptionskapazität des oder der basischen Feststoffe(s) erschöpft ist, kann er/können sie durch Behandlung mit Sauerstoff oder sauerstoffhaltigen Gasgemischen bei erhöhter Temperatur von bevorzugt 250 bis 500 °C unter Wiedergewinnung der ursprünglichen Adsorptionskapazität regeneriert werden. Bei Verwendung eines Festbetts mit ausreichend großer Kapazität erfolgt die Regeneration bevorzugt zusammen mit der Regeneration des Hydrierkatalysators. Bei Betrieb mehrerer Festbetten im Wechsel wird jeweils ein Festbett für die Adsorption genutzt, und die anderen werden währenddessen regeneriert. Ist der basische Feststoff mit dem Träger der hydrieraktiven Metalle identisch bzw. auf diesem aufgebracht, so wird seine Adsorptionskapazität bei der Regeneration des Hydrierkatalysators wiederhergestellt..

Zur Erleichterung der Regeneration können die basischen Feststoffe mit geeigneten Oxidationskatalysatoren versehen werden. Hier kommen Metalle bzw. Metalloxide der Gruppen 5 - 7 bzw. 10 und 11 des Periodensystems der Elemente (Nomenklatur nach IUPAC-Empfehlung von 1986) in Frage, bevorzugt sind Oxide des Vanadiums. Genannte Oxidationskatalysatoren liegen in Mengen von 0,1 bis zu 10 Massenprozent, bezogen auf die Gesamtmasse des basischen Feststoffs (einschließlich Oxidationskatalysator), vor.

Weitere Aufarbeitungsschritte, wie z. B. Destillations- oder Waschstufen, können zur Erreichung noch höherer Reinheitsgrade des aromatischen Amins nachgeschaltet werden, sind aber nicht zwingend erforderlich. Die nachgeschalteten Wasch- und/oder Destillationsschritte können in allen Fachleuten geläufigen Varianten ausgestaltet und unter verschiedensten Bedingungen betrieben werden. So kann eine Destillation z. B. in einer oder mehreren Glockenboden- oder Packungskolonnen, aber auch in Trennwandkolonnen erfolgen. Dabei können Leichtsieder und Schwersiederabtrennung in verschiedenen Kolonnen, aber auch gemeinsam in einer Kolonne unter Seitenstromentnahme des Anilins erfolgen.

Die in dem erfindungsgemäßen Verfahren erhältlichen gereinigten aromatischen Amine enthalten in der Summe, bezogen auf die Masse des Amins, bevorzugt weniger als 0,01 Massen-%, besonders bevorzugt weniger als 0,005 Massen-%, ganz besonders bevorzugt weniger als 0,002 Massen-% an phenolischen Verbindungen. Unter "phenolischen Verbindungen" ist die Summe aller Verbindungen mit phenolischen Hydroxygruppen und ihrer Metallsalze zu verstehen.

Die nach dem erfindungsgemäßen Verfahren erhaltenen aromatischen Amine sind aufgrund ihrer hohen Reinheit für eine weitere großtechnische Verwendung besonders geeignet. Beispielsweise kann nach dem erfindungsgemäßen Verfahren gereinigtes Anilin nach den aus dem Stand der Technik bekannten Methoden mit Formaldehyd in Gegenwart eines sauren Katalysators zu Di- und Polyaminen der Diphenylmethanreihe umgesetzt werden. Die so gewonnenen Di- und Polyamine können anschließend nach den aus dem Stand der Technik bekannten Verfahren, z. B. mit Phosgen, zu den entsprechenden Di- und Polyisocyanaten der Diphenylmethanreihe umgesetzt werden.

**Beispiele**

Im Folgenden sind Beispiele für die Durchführung des erfindungsgemäßen Verfahrens gegeben. Die Analyse der Phenolgehalte in den nachfolgenden Beispielen erfolgt mittels Gaschromatographie.

**Beispiel 1**

Durch ein von außen auf 240 °C beheiztes Adsorberrohr von 60 cm Länge und 2,5 cm Durchmesser, gefüllt mit Glaskugeln und einer Schüttung aus 14 g des Adsorbens, wird bei Umgebungsdruck ein gasförmiger Strom aus 49,12 g/h Anilin (dotiert mit 500 ppm Phenol), 20,1 g/h Wasser und 24,5 1/h Wasserstoff geleitet. Der aus dem Adsorber austretende Gasstrom wird bei 3 °C in einem Auffangbehälter kondensiert und der Phenolgehalt gaschromatographisch bestimmt. Die Adsorptionsdauer beträgt 6 h. Tabelle 1 enthält die gefundenen Adsorptionskapazitäten und -selektivitäten.

**Tabelle 1**

| Adsorbens | Adsorbierte Menge in $g_{Phenol}/kg_{Adsorbens}$ | Adsorptionsselektivität A in % |
|---|---|---|
| $Al_2O_3$ (Saint Gobain NorPro) | 4,30 | 99,60 |
| MgO (Engelhard) | 2,60 | 99,64 |
| MgO / $V_2O_5$ | 4,20 | Nicht bestimmt. |

**Beispiel 2**

Durch das in Beispiel 1 genannte Adsorberrohr, gefüllt mit Glaskugeln und einer Schüttung aus 10 g Adsorbens, wird bei Umgebungsdruck und 240 °C ein gasförmiger Strom aus 8 g/h Anilin (dotiert mit 500 ppm Phenol), 3,2 g/h Wasser und 5,76 1/h Wasserstoff geleitet. Der aus dem Adsorber austretende Gasstrom wird bei 3 °C in einem Auffangbehälter kondensiert und der Phenolgehalt gaschromatographisch bestimmt. Tabelle 2 fasst die gefundenen Phenolgehalte zusammen.

**Tabelle 2**

| | Phenolgehalt in ppm | |
|---|---|---|
| Zeit t in min | Hydrotalcit Kyowaad 500 (Kyowa Chemical) | MgO (Engelhard) |
| 30 | 7,5 | 0,0 |
| 60 | 7,4 | 0,0 |
| 90 | 7,2 | 10,9 |
| 120 | 8,2 | 10,8 |
| 150 | 7,7 | 10,5 |
| 180 | 7,2 | 10,6 |
| 210 | 7,2 | 0,0 |
| 240 | 6,8 | 10,6 |
| 270 | 7,3 | 17,9 |
| 300 | 7,5 | 10,5 |

**Beispiel 3**

Ein beladenes Adsorbens aus Beispiel 1 wird regeneriert, indem bei 340 °C und Umgebungsdruck alle adsorbierten organischen Verbindungen in einem Strom aus Luft in Stickstoff abgebrannt werden. Die Adsorbenzien aus Beispiel 1 können ohne signifikanten Aktivitätsverlust jeweils 5 x regeneriert und wieder beladen werden.

**Patentansprüche**

1. Verfahren zur Entfernung von Verbindungen mit phenolischen Hydroxygruppen aus einem mindestens ein aromatisches Amin enthaltenden Gasstrom, welcher aus der Gasphasen-Hydrierung der entsprechenden aromatischen Nitroverbindungen mit Wasserstoff hervorgeht, bei dem die Verbindungen mit phenolischen Hydroxygruppen an einem basischen Feststoff adsorbiert werden.

2. Verfahren nach Anspruch 1, worin der über den basischen Feststoff geleitete Gasstrom eine Eingangstemperatur zwischen 150 und 500 °C und einen absoluten Druck von 1 bis 50 bar aufweist.

3. Verfahren nach Anspruch 1 oder 2, worin der basische Feststoff Oxide und Hydroxyde von Elementen der Gruppen 1 bis 2 bzw. 12 bis 14 des Periodensystems der Elemente enthält.

4. Verfahren nach Anspruch 1 oder 2, worin der basische Feststoff Zeolithe enthält.

5. Verfahren nach einem der Ansprüche 1 bis 4, worin der basische Feststoff 0,1 -10 Massen-% eines Oxidationskatalysators, bezogen auf die Gesamtmasse des basischen Feststoffs einschließlich Oxidationskatalysator, enthält.

6. Verfahren nach Anspruch 5, bei dem der Oxidationskatalysator Metalle bzw. Metalloxide aus den Gruppen 5 bis 7 bzw. 10 bis 11 des Periodensystems der Elemente enthält.

7. Verfahren nach einem der Ansprüche 1. bis 6, worin der basische Feststoff nach vollständiger oder teilweiser Beladung mit den Verbindungen mit phenolischen Hydroxygruppen durch Behandlung mit Sauerstoff oder sauerstoffhaltigen Gasgemischen bei Temperaturen von 250 bis 500 °C regeneriert wird.

8. Verfahren nach einem der Ansprüche 1 bis 7, worin die Adsorption entweder in einem Festbett oder unter Betrieb zweier oder mehrerer parallel verschalteter Festbetten im Wechsel erfolgt.

9. Verfahren nach einem der Ansprüche 1 bis 8, worin die Gasphasen-Hydrierung der Nitroverbindung in Gegenwart eines ortsfesten, heterogenen Trägerkatalysators durchgeführt wird, und der basische Feststoff identisch mit dem Träger ist oder auf dem Träger aufgebracht ist, der als Katalysatorträger in der Gasphasen-Hydrierung der Nitroverbindung eingesetzt wird.

**Claims**

1. Process for removing compounds having phenolic hydroxy groups from a gas stream that contains at least one aromatic amine and that arises from gas-phase hydrogenation of the corresponding aromatic nitro compounds with hydrogen, wherein the compounds having phenolic hydroxy groups are adsorbed on a basic solid.

2. Process according to Claim 1, in which the gas stream passed over the basic solid exhibits an inlet temperature between 150 °C and 500 °C and an absolute pressure from 1 bar to 50 bar.

3. Process according to Claim 1 or 2, in which the basic solid contains oxides and hydroxides of elements pertaining to Groups 1 to 2 and 12 to 14, respectively, of the periodic system of the elements.

4. Process according to Claim 1 or 2, in which the basic solid contains zeolites.

5. Process according to one of Claims 1 to 4, in which the basic solid contains 0.1 wt.% to 10 wt.% of an oxidation catalyst, relative to the total weight of the basic solid including oxidation catalyst.

6. Process according to Claim 5, in which the oxidation catalyst contains metals or oxides of metals from Groups 5 to 7 or 10 to 11, respectively, of the periodic system of the elements.

7. Process according to one of Claims 1 to 6, in which after total or partial loading with the compounds having phenolic hydroxy groups the basic solid is regenerated by treatment with oxygen or oxygen-containing gas mixtures at temperatures from 250 °C to 500 °C.

8. Process according to one of Claims 1 to 7, in which the adsorption takes place either in one fixed bed or by operation in alternation of two or more fixed beds connected in parallel.

9. Process according to one of Claims 1 to 8, in which the gas-phase hydrogenation of the nitro compound is carried out in the presence of a fixed, heterogeneous supported catalyst, and the basic solid is identical with the support or is applied to the support that is used as catalyst support in the gas-phase hydrogenation of the nitro compound.

**Revendications**

1. Procédé d'élimination des composés avec radicaux hydroxyle phénoliques, d'un courant gazeux contenant au moins une amine aromatique, qui provient de l'hydrogénation en phase gazeuse des composés nitro aromatiques correspondants, avec de l'hydrogène, où les composés avec radicaux hydroxyle phénoliques sont adsorbés sur un solide basique.

2. Procédé selon la revendication 1, où le courant gazeux dirigé sur le solide basique présente une température d'entrée allant de 150 à 500°C et une pression absolue allant de 1 à 50 bar.

3. Procédé selon la revendication 1 ou 2, où le solide basique contient un oxyde ou hydroxyde des éléments des groupes 1 à 2 et respectivement, 12 à 14 du système périodique des éléments.

4. Procédé selon la revendication 1 ou 2, où le solide basique contient une zéolite.

5. Procédé selon l'une des revendications 1 à 4, où le solide basique contient 0,1-10% en masse d'un catalyseur d'oxydation, par rapport à la masse totale du solide basique y compris le catalyseur d'oxydation.

6. Procédé selon la revendication 5, où le catalyseur d'oxydation contient des métaux et respectivement, des oxydes métalliques des groupes 5 à 7 et respectivement, 10 à 11 du système périodique des éléments.

7. Procédé selon l'une des revendications 1 à 6, où le solide basique est régénéré après charge complète ou partielle avec les composés avec radicaux hydroxyle phénoliques, par traitement avec de l'oxygène ou des mélanges gazeux contenant de l'oxygène, à des températures allant de 250 à 500°C.

8. Procédé selon l'une des revendications 1 à 7, où l'adsorption est réalisée dans un lit fixe ou par utilisation de deux ou plusieurs lits fixes disposés en parallèle, alternativement.

9. Procédé selon l'une des revendications 1 à 8, où l'hydrogénation en phase gazeuse du composé nitro est réalisée en présence d'un catalyseur supporté hétérogène, fixe, et le solide basique est identique au support ou est appliqué sur le support, qui est mis en oeuvre comme support de catalyseur dans l'hydrogénation en phase gazeuse du composé nitro.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2201528 A
- DE 3414714 A
- US 3136818 A
- EP 0696573 B1
- EP 0696574 B1
- JP 49035341 A
- JP 8295654 A

**In der Beschreibung aufgeführte Nicht-Patentliteratur**

- **REP; M. ; PALOMARES, A. E. ; EDER-MIRTH, G. ; VAN OMMEN, J. G. ; RÖSCH, N. ; LERCHER, J. A.** Interaction of Methanol with Alkali Metal Exchanged Molecular Sieves. 1. IR Spectroscopic Study. *Journal of Physical Chemistry B,* 2000, vol. 104 (35), 8624-8630
- **TSYGANENKO, A. A. ; KONDRATIEVA, E. V. ; YANKO, V. S. ; STOROZHEV, P. YU.** FTIR study of CO adsorption on basic zeolites. *Journal of Materials Chemistry,* 2006, vol. 16 (24), 2358-2363
- **PLANT, DAVID F. ; SIMPERLER, ALEXANDRA ; BELL, ROBERT G.** Adsorption of methanol on zeolites X and Y. An atomistic and quantum chemical study. *Journal of Physical Chemistry B,* 2006, vol. 110 (12), 6170-6178
- **WIBERG, N.** Hollemann-Wiberg - Lehrbuch der Anorganischen Chemie. 1995, vol. 101, 939